# EUROPEAN PATENT APPLICATION

(11) **EP 4 471 153 A1**
(43) Date of publication of application: **04.12.2024**
(21) Application number: 23747082.8
(22) Date of filing: 27.01.2023
(51) Int. Cl.: C12Q 1/26, C12Q 1/68

(54) **METHOD AND COMPOSITION FOR STABILIZING NICOTINAMIDE ADENINE DINUCLEOTIDE**

(30) Priority: 28.01.2022 JP 2022011518
(71) Applicant: Oriental Yeast Co., Ltd., Tokyo 174-8505 (JP)
(72) Inventor: TAKETANI, Yukiko, Nagahama-shi, Shiga 526-0804 (JP); MATSUKAWA, Hirokazu, Nagahama-shi, Shiga 526-0804 (JP); SUNAHARA, Yoshiko, Nagahama-shi, Shiga 526-0804 (JP)
(74) Representative: Hoffmann Eitle
(86) International application number: PCT/JP2023/002617
(87) International publication number: WO 2023/145872

(57) **Abstract**

Method and composition of stabilizing nicotinamide adenine dinucleotide (NAD) in a biological sample are provided. NAD in the sample is stabilized by contacting the sample collected from a subject with nicotinamide and/or nicotinamide derivative.

## Description

### Technical Field

The present invention relates to a method and a composition for stabilizing nicotinamide adenine dinucleotide, and a method and a kit for measuring nicotinamide adenine dinucleotide.

### Background Art

Nicotinamide adenine dinucleotide (NAD) is a pyridine nucleotide that functions as an electron carrier in a cellular metabolism of aerobic respiration in many of major oxidation-reduction reactions in living bodies, and plays an important role in maintaining an oxidation-reduction homeostatic balance. As shown in Non Patent Literatures 1 and 2, NAD in human tissues and plasma is known to decrease with aging. Moreover, Patent Literature 1 discloses that the amount of metabolites such as NAD⁺ in blood can be used as an indicator for determining the degree of human aging.

As a method of measuring NAD concentration in a biological sample, Non Patent Literature 3 discloses a simple quantitative method using a microplate reader. It discloses that in this method, a blood sample for NAD measurement is stable for 5 hours after being collected from a subject, suspended in a buffer solution for extraction, and cooled in ice. In other words, to ensure sample stability, it shows that it is not preferable to place a biological sample at ordinary temperature or in ice for an extended period of time.

### Citation List

### Patent Literature

Patent Literature 1: JP Patent Publication (Kohyo) No. 2019-509489

### Non Patent Literature

Non Patent Literature 1: Hassina Massudi et al., PLoS One. 2012; 7(7): e42357
Non Patent Literature 2: James Clement et al., Rejuvenation Res 2019 Apr; 22(2): 121-130
Non Patent Literature 3: Katsumi Shibata et al., Vitamins, 2001(September); Vol. 75, No. 9: 455-462

### Summary of Invention

### Technical Problem

NAD in a biological sample is highly unstable, and it is a problem that a sample cannot be stored for a long time even under refrigerated condition. Especially for a whole blood sample, it is necessary to immediately deproteinize fresh blood after collection. In practice, however, it is often difficult to immediately measure NAD in a biological sample or to immediately deproteinize a biological sample at a clinical site where it is collected. Therefore, a simple method is desirable to stabilize NAD in a biological sample.

The purpose of the present invention is to provide a method and a composition for stabilizing NAD in a biological sample. Another purpose of the present invention is to provide an NAD measurement method and an NAD measurement kit to measure the amount of NAD in a biological sample while at a stable NAD content.

### Solution to Problem

The present inventors conducted intensive studies and have found that the NAD concentration in a biological sample is stabilized by contacting the NAD in a biological sample with nicotinamide and/or nicotinamide derivative. On the basis of the findings, the present inventors have completed the present invention.

In other words, the present invention provides the following.
(1) A method of stabilizing nicotinamide adenine dinucleotide (NAD) in a sample collected from a subject, comprising
   a contact step of contacting the sample with nicotinamide and/or nicotinamide derivative.
(2) The method according to (1), wherein the nicotinamide and/or nicotinamide derivative is nicotinamide, nicotinamide mononucleotide (NMN) and/or nicotinamide riboside (NR).
(3) The method according to (1) or (2), wherein the nicotinamide and/or nicotinamide derivative comprises two or more compounds selected from the group consisting of nicotinamide, NMN and NR.
(4) The method according to (2) or (3), wherein in the contact step:
   i) a final concentration of the nicotinamide is 10 to 500 mM;
   ii) a final concentration of the NMN is 1 to 100 mM; and/or
   iii) a final concentration of the NR is 1 to 100 mM.
(5) The method according to any of (1) to (4), wherein the sample is whole blood or tissue fluid from the subject.
(6) The method according to any of (1) to (5), wherein the subject is human.
(7) A method of measuring NAD, comprising a step of measuring NAD in a sample stabilized by the method according to any of (1) to (6).
(8) The method according to (7), further comprising a step of pretreating a sample before the step of measuring NAD.
(9) The method according to (8), wherein the step of measuring NAD includes reacting a pretreated sample in a system comprising dehydrogenase with NAD⁺ as a coenzyme and a substrate thereof, diaphorase, and a tetrazolium salt.
(10) A composition comprising nicotinamide and/or nicotinamide derivative, for use in stabilizing NAD in a sample.
(11) The composition according to (10), wherein the nicotinamide and/or nicotinamide derivative is nicotinamide, NMN and/or NR.
(12) The composition according (10) or (11), wherein the nicotinamide and/or nicotinamide derivative comprises two or more compounds selected from the group consisting of nicotinamide, NMN and NR.
(13) The composition according to any of (10) to (12), wherein the composition is an aqueous solution.
(14) The composition according to any of (10) to (13), for use in stabilizing NAD in a sample collected from a subject.
(15) The composition according to any of (10) to (14), wherein the sample is whole blood or tissue fluid from the subject.
(16) The composition according to any of (10) to (15), wherein the subject is human.
(17) The composition according to any of (10) to (16), for use in the method according to any of (1) to (6).
(18) An NAD measurement kit comprising the composition according to any of (10) to (17) and a reagent for measuring NAD in a sample.
(19) The NAD measurement kit according to (18), further comprising a pretreatment reagent for pretreating a sample.
(20) The NAD measurement kit according to (19), wherein the reagent for measuring NAD comprises a first test solution comprising dehydrogenase with NAD⁺ as a coenzyme and a tetrazolium salt, and a second test solution comprising a substrate of the dehydrogenase and diaphorase.

The description includes the disclosure content of Japanese Patent Application No. 2022-011518, that is a basis of priority of the application.

### Advantageous Effects of Invention

The present invention provides a method and a composition for stabilizing NAD in a biological sample. The present invention further provides an NAD measurement method and an NAD measurement kit to measure the amount of NAD in a biological sample at a stable NAD content.

### Brief Description of Drawings

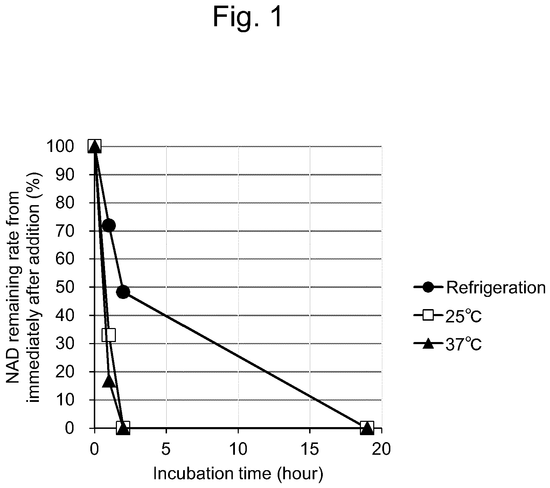

[Figure 1] The line graph illustrates a change of the NAD content after NAD was added into pooled human serum during incubation at refrigeration, 25°C and 37°C. The value is a relative value (%) of NAD concentration in serum under each incubation condition to the concentration at the time of NAD addition (0 hour) that is set as 100%.

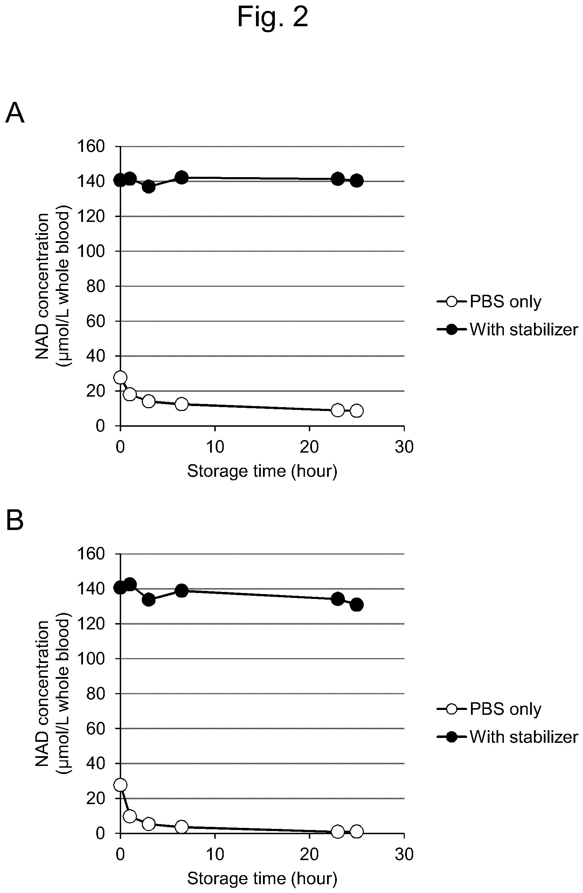

[Figure 2] The line graph illustrates a change of the NAD content in a sample of rat whole blood with NAD stabilizer or PBS at refrigeration and 25°C. Figure A is a graph illustrating the time-course change of NAD concentration in a sample under a static condition at refrigeration. Figure B is a graph illustrating the time-course change of NAD concentration in a sample under a static condition at 25°C.

### Description of Embodiments

### 1. Nicotinamide adenine dinucleotide (NAD) stabilization methods

### 1-1. Constitution

The first embodiment in the present invention provides a method of stabilizing nicotinamide adenine dinucleotide (NAD) in a biological sample collected from a subject (hereinafter also referred to as "NAD stabilization method"). The NAD stabilization method in the present embodiment comprises a contact step of contacting a biological sample collected from a subject with nicotinamide and/or nicotinamide derivative. The NAD stabilization method in the embodiment can suppress a decrease of NAD amount in a biological sample when nicotinamide and/or nicotinamide derivative coexist with NAD.

### 1-2. Definition

In the description, "subject" refers to mammals. Mammals refer to animals belonging to the class *Mammalia* of the subphylum *Vertebrata* of the phylum *Chordata,* including humans and non-humans, and include, for example, humans, primates such as chimpanzees, pet animals such as dogs and cats, livestock animals such as cattle, pigs, horses, sheep, and goats, rodents such as mice and rats, and mammals raised in zoos. The subject in the description is preferably human.

In the description, "sample" or "biological sample" is not particularly limited. For example, the sample can be a sample collected from a subject including blood (e.g., whole blood, serum, plasma), urine, feces, saliva, milk, tissue fluid or cell extracts, hair, sweat, or a mixture thereof. A preferred biological sample in the description is whole blood or tissue fluid. As used herein, "whole blood" refers to whole blood that has been mixed with an anticoagulant immediately after collection so that blood coagulation does not occur, unless otherwise specified. The anticoagulant is usually dipotassium EDTA, disodium EDTA, heparin, or trisodium citrate, but any anticoagulant may be used.

In the description, nicotinamide adenine dinucleotide (NAD) refers to an electron carrier that has a structure in which nicotinamide mononucleotide and adenosine are bound via a phosphate bond and functions as a coenzyme for oxidoreductase. NAD has an oxidized form (NAD⁺) and a reduced form (NADH) represented by the following formula (I), and the NAD according to the description includes both the oxidized form and the reduced form.

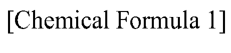

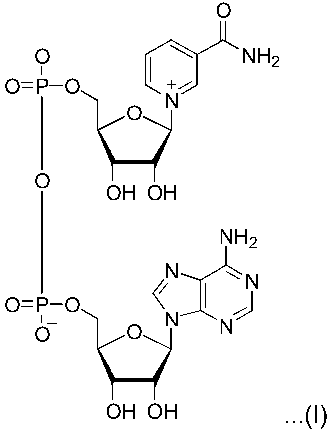

The conversion reaction from the oxidized form to the reduced form, and the reduced form to the oxidized form, are converted by reactions occurring at the site of nicotinic acid amide, as shown in the following formula (II). This conversion reaction is reversible.

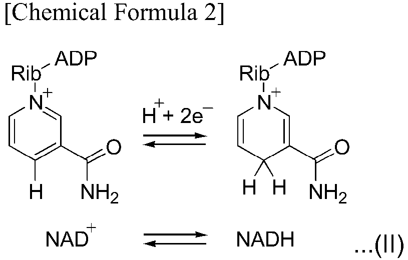

In the description, "nicotinamide" refers to a compound represented by the following formula (III).

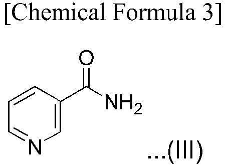

In the description, "nicotinamide derivative" refers to a compound having a nicotinamide skeleton represented by formula (III). The compound is not particularly limited as long as it is a compound that has a nicotinamide skeleton. Examples of the compound include nicotinamide mononucleotide (NMN) and nicotinamide riboside (NR), and they include both an oxidized form and a reduced form.

An oxidized form of nicotinamide mononucleotide (NMN) has a structure represented by the following formula (IV).

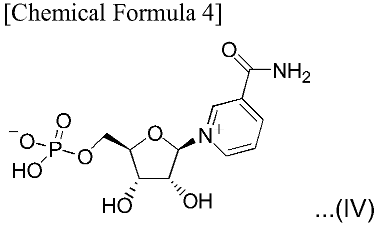

An oxidized form of nicotinamide riboside (NR) has a structure represented by the following formula (V).

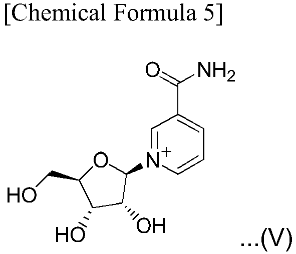

### 1-3. Contact step

The NAD stabilization method in the embodiment includes a contact step, wherein a sample to be measured for NAD is contacted with nicotinamide and/or nicotinamide derivative (hereinafter also referred to as "nicotinamides"). The method of bringing nicotinamides into contact with a sample is not particularly limited. Examples of the method include, in the case of a liquid sample, dissolving a solid composition in a solid form, a powder form, and the like comprising nicotinamides in a sample, or mixing an aqueous solution composition comprising nicotinamides with a sample. In particular, it is preferable to employ a method of mixing an aqueous solution composition with a sample.

The nicotinamides to be used are not particularly limited as long as they are a compound having a nicotinamide skeleton. However, it is particularly preferred to be nicotinamide, nicotinamide mononucleotide (NMN) and/or nicotinamide riboside (NR). Further, it is more preferred to be two or more compounds selected from the group consisting of nicotinamide, NMN and NR. For example, a mixture of nicotinamide and NMN, a mixture of nicotinamide and NR, or a mixture of NMN and NR can be used.

When nicotinamide is used as nicotinamides, the final concentration of the nicotinamide when mixed with a sample is preferably 10 to 500 mM. The lower limit of the final concentration of the nicotinamide can be not less than 10 mM, not less than 20 mM, not less than 30 mM, not less than 40 mM, not less than 50 mM, not less than 60 mM, not less than 70 mM, not less than 80 mM, not less than 90 mM, not less than 100 mM, not less than 150 mM, not less than 200 mM, not less than 250 mM, not less than 300 mM. Moreover, the upper limit of the final concentration of the nicotinamide can be not more than 500 mM, not more than 450 mM, not more than 400 mM, not more than 350 mM, not more than 300 mM, not more than 250 mM, not more than 200 mM, not more than 150 mM, not more than 100 mM, not more than 90 mM, not more than 80 mM, not more than 70 mM, not more than 60 mM, not more than 50 mM. When NMN is used as nicotinamides, the final concentration of the NMN when mixed with a sample is preferably 1 to 100 mM. The lower limit of the final concentration of the NMN can be not less than 1 mM, not less than 2 mM, not less than 3 mM, not less than 4 mM, not less than 5 mM, not less than 6 mM, not less than 7 mM, not less than 8 mM, not less than 9 mM, not less than 10 mM, not less than 15 mM, not less than 20 mM, not less than 25 mM, not less than 30 mM, not less than 40 mM, not less than 50 mM, not less than 60 mM, not less than 70 mM, not less than 80 mM, not less than 90 mM. Moreover, the upper limit of the final concentration of the NMN can be not more than 100 mM, not more than 90 mM, not more than 80 mM, not more than 70 mM, not more than 60 mM, not more than 50 mM, not more than 45 mM, not more than 40 mM, not more than 35 mM, not more than 30 mM, not more than 25 mM, not more than 20 mM, not more than 15 mM, not more than 10 mM, not more than 9 mM, not more than 8 mM, not more than 7 mM, not more than 6 mM, not more than 5 mM. When NR is used as nicotinamides, the final concentration of the NR when mixed with a sample is preferably 1 to 100 mM. The lower limit of the final concentration of the NR can be not less than 1 mM, not less than 2 mM, not less than 3 mM, not less than 4 mM, not less than 5 mM, not less than 6 mM, not less than 7 mM, not less than 8 mM, not less than 9 mM, not less than 10 mM, not less than 15 mM, not less than 20 mM, not less than 25 mM, not less than 30 mM, not less than 40 mM, not less than 50 mM, not less than 60 mM, not less than 70 mM, not less than 80 mM, not less than 90 mM. The upper limit of the final concentration of the NR can be not more than 100 mM, not more than 90 mM, not more than 80 mM, not more than 70 mM, not more than 60 mM, not more than 50 mM, not more than 45 mM, not more than 40 mM, not more than 35 mM, not more than 30 mM, not more than 25 mM, not more than 20 mM, not more than 15 mM, not more than 10 mM, not more than 9 mM, not more than 8 mM, not more than 7 mM, not more than 6 mM, not more than 5 mM.

By using the NAD stabilization method in the embodiment, it was confirmed that the NAD amount in a biological sample such as a whole blood sample is stable for at least 24 hours under a storage condition at refrigeration or room temperature. Since the method in the embodiment can be achieved by a simple method including mixing a composition comprising nicotinamides with a collected sample, it can be performed in a clinical site immediately after the sample is collected (blood collection). Therefore, the method enables the transport of a biological sample such as whole blood for measuring NAD from a clinical site to a laboratory under refrigeration or room temperature storage conditions for NAD measurement at a laboratory.

### 2. NAD measurement methods

The second embodiment of the present invention provides an NAD measurement method. The NAD measurement method in the present embodiment comprises a step of measuring NAD in a sample stabilized using the method according to section "1. Nicotinamide adenine dinucleotide (NAD) stabilization methods." The NAD measurement method in the embodiment enables more accurate quantitation of NAD amount in living bodies. Since the NAD amount in living bodies can be an indicator of the degree of aging, the degree of aging of a subject can be determined with higher accuracy. In the embodiment, the definitions of terms are the same as those according to section "1. Nicotinamide adenine dinucleotide (NAD) stabilization methods" unless otherwise specified.

The embodiment comprises a step of measuring NAD in a sample. Preferably, the embodiment comprises a step of pretreating a stabilized sample using the method according to section "1. Nicotinamide adenine dinucleotide (NAD) stabilization methods" before a step of measuring NAD in the sample. The pretreatment step for a sample can be, for example, performing a deproteinization treatment. Any known methods of deproteinizing treatment can be used, such as a deproteinizing treatment using a protein denaturation and precipitation method. In the description, the protein denaturation and precipitation method refer to a method of adding a protein precipitant comprising an organic solvent such as ethanol, acetone, or acetonitrile, or an acid such as trichloroacetic acid or perchloric acid to a sample, and the mixture is then mixed, cooled, and centrifuged to collect its supernatant. Since NAD is particularly susceptible to degradation by the effect of proteins derived from a sample, performing the deproteinization treatment is preferred.

In a step of measuring NAD in a sample, preferably a pretreated sample, a method of measuring NAD is not particularly limited and any known methods such as gas chromatography-mass spectrometry (GC/MS), liquid chromatography-mass spectrometry (LC/MS), competitive immunoassay, and enzymatic cycling can be used.

When using an enzymatic cycling method, for example, a pretreated sample can be reacted in a system comprising dehydrogenase with NAD⁺ as a coenzyme (e.g., glucose dehydrogenase), a substrate thereof (e.g., glucose), diaphorase, and a tetrazolium salt (e.g., WST-8). In the system, an enzymatic cycling reaction represented by the following formula (VI) occurs.

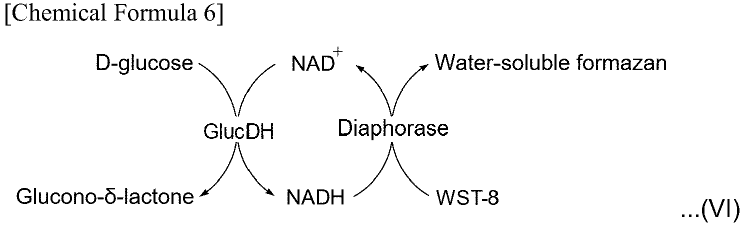

In the reaction represented by formula (VI), NAD⁺ is reduced to produce NADH by the reaction between glucose and glucose dehydrogenase in the presence of NAD⁺. On the other hand, NADH is oxidized to produce NAD⁺ by the reaction between diaphorase and WST-8 in the presence of NADH. Both enzymatic reactions result in a cycling reaction of NAD⁺ and NADH. The absorbance (at the wavelength near 450 nm that is the maximum absorption wavelength) of water-soluble formazan produced by the reduction of WST-8 is measured over time to calculate an increase rate in absorbance. By performing the similar reaction in advance using a standard solution comprising NAD at a predetermined concentration, a calibration curve can be created using the NAD concentrations and an increase rate in absorbance. The NAD concentration of a sample can be calculated based on a rate of absorbance obtained from the sample.

The dehydrogenase with NAD⁺ as a coenzyme is not particularly limited as long as it is the enzyme with NAD⁺ as a coenzyme. For example, glucose dehydrogenase, lactate dehydrogenase, and alcohol dehydrogenase can be used. When glucose dehydrogenase is used, the reaction concentration is preferably 50 to 5000 U/L, particularly 100 to 3000 U/L, or 250 to 2000 U/L.

Diaphorase (hereinafter also referred to as NADH dehydrogenase) is known as an enzyme that uses NADH to reduce a tetrazolium salt to produce formazan and simultaneously oxidizes NADH to produce NAD⁺. The enzyme is not particularly limited in terms of species, structure, and the like as long as it is an enzyme that produces those reactions. For example, diaphorase derived from *Clostridium kluyveri* can be used. The reaction concentration of diaphorase is preferably 100 to 5000 U/L, particularly 200 to 3000 U/L, or 500 to 2000 U/L.

Tetrazolium salt that is a substrate of diaphorase is not particularly limited as long as it produces water-soluble formazan in the reaction with the diaphorase. For example, 2-(2-methoxy-4-nitrophenyl)-3-(4-nitrophenyl)-5-(2,4-disulfophenyl)2H-tetrazolium monosodium salt (also referred to as WST-8), 2-(4-indophenyl)-3-(4-nitrophenyl)-5-(2,4-disulfophenyl)-2H-tetrazolium monosodium salt, and 2-(4- indophenyl)-3-(2,4-dinitrophenyl)-5-(2,4-disulfophenyl)-2H-tetrazolium monosodium salt can be used.

The enzymatic cycling method described above can be performed simply, for example, by using an existing automated clinical laboratory analyzer (e.g., 7180 type Hitachi automatic analyzer (manufactured by Hitachi High-Tech Corporation)). Alternatively, the enzymatic cycling method described above can be replaced by the method using a microplate reader according to Non Patent Literature 3.

### 3. NAD stabilizing compositions

The third embodiment of the present invention is a composition to be used for stabilizing NAD in a biological sample (hereinafter also referred to as "NAD stabilizing composition"). The NAD stabilizing composition of the embodiment comprises nicotinamide and/or nicotinamide derivative. According to the NAD stabilizing compositions in the embodiment, it is possible to stabilize the NAD concentration in a sample by bringing the composition into contact (mixing, dissolving) with the sample.

The NAD stabilizing composition in the embodiment can be used in the NAD stabilization method according to section "1. Nicotinamide adenine dinucleotide (NAD) stabilization methods." In the embodiment, the definitions of terms are the same as those according to section "1. Nicotinamide adenine dinucleotide (NAD) stabilization methods" unless otherwise specified.

The NAD stabilizing composition in the embodiment comprises nicotinamide and/or nicotinamide derivative (nicotinamides) as an active ingredient, and the composition is not particularly limited in terms of properties and the like, as long as it is possible to contact a sample with the active ingredient. For example, the composition may be a solid composition in a solid form, a powder form, and the like. Further, the composition may be a composition of an aqueous solution comprising nicotinamides. In particular, it is preferable to employ an aqueous solution that can be easily mixed with a sample.

Nicotinamides that are comprised in an NAD stabilizing composition are not particularly limited as long as they are compounds that have a nicotinamide skeleton. Particularly, however, the compound is preferably nicotinamide, NMN and/or NR. Further, it is more preferred to be two or more compounds selected from the group consisting of nicotinamide, NMN and NR. For example, a mixture of nicotinamide and NMN, a mixture of nicotinamide and NR, or a mixture of NMN and NR can be used.

The amount of nicotinamides comprised in an NAD stabilizing composition can be adjusted to achieve a desired final concentration when the NAD stabilizing composition is brought in contact (e.g., mixed, dissolved) with a sample. For example, when the composition is an aqueous solution, a sample and a composition may be mixed at a 9:1 volume ratio, with the nicotinamide concentration in the composition at 10 times the desired final concentration. In this case, the concentration of nicotinamides in the composition can be, for example, 100 to 5000 mM for nicotinamide, 10 to 1000 mM for NMN, and 10 to 1000 mM for NR.

When an NAD stabilizing composition is an aqueous solution, the composition may comprise a pH buffer such as phosphoric acid, citric acid, Tris, MES, HEPES, or PIPES, a chelating agent such as EDTA, a surfactant, an antioxidant, an antiseptic, or the like as needed, in addition to nicotinamides as an active ingredient. The pH of the composition as an aqueous solution is preferably pH 3.0 to 8.0, and more preferably pH 4.0 to 7.0, in consideration of the stability of nicotinamides.

When an NAD stabilizing composition is solid (e.g., solids, powder), the composition may comprise solubilizers, pH buffers, chelating agents, antioxidants, antiseptics, bulking agents, excipients, and the like as needed, in addition to nicotinamides as an active ingredient. The pH of the composition as a solid is preferably adjusted, when the composition is mixed with a biological sample, to pH 3.0 to 8.0, particularly pH 4.0 to 7.0.

### 4. NAD measurement kits

The fourth embodiment in the present invention is an NAD measurement kit. The NAD measurement kit in the embodiment comprises the composition according to section "3. NAD stabilizing compositions" and a reagent for measuring NAD. As used herein, the reagent for measuring NAD is preferably the reagent for use in the NAD measurement method according to section "2. NAD measurement methods". In the embodiment, the definitions of terms are the same as those according to section "1. Nicotinamide adenine dinucleotide (NAD) stabilization methods" unless otherwise specified.

In the embodiment, the NAD measurement kit preferably further comprises a pretreatment reagent for pretreating a sample. For example, a reagent for deproteinizing a sample can be used as a pretreatment reagent. The protein denaturation and precipitation method can be used as the deproteinization treatment, wherein a protein precipitant can be used as the pretreatment reagent. Examples of the protein precipitant include organic solvents such as ethanol, acetone, and acetonitrile and acids such as trichloroacetic acid and perchloric acid.

In the embodiment, the "reagent for measuring NAD" (hereinafter also referred to as "NAD measurement reagent") is not particularly limited in terms of measurement principles and the like, as long as the reagent is a reagent that can perform quantitation of NAD. An NAD measurement reagent that is suitable for known methods can be used. Examples of the known methods include gas chromatography-mass spectrometry (GC/MS), liquid chromatography-mass spectrometry (LC/MS), competitive immunoassay, and enzymatic cycling. Preferably, an NAD measurement reagent that is suitable for an enzymatic cycling method can be used.

With the enzymatic cycling method, for example, the principles according to formula (VI) in section "2. NAD measurement methods" can be used. When the principles according to formula (VI) are used, the NAD measurement reagent preferably comprises the following test solutions:
first test solution: dehydrogenase with NAD⁺ as a coenzyme (glucose dehydrogenase according to formula (VI)) and tetrazolium salt (WST-8 according to formula (VI)); and
second test solution: a substrate of the dehydrogenase (D-glucose according to formula (VI)) and diaphorase.

The first test solution is mixed with a sample comprising NAD, and the second test solution is added thereto to react. An enzyme cycling reaction occurs according to formula (VI). The reaction reduces the tetrazolium salt to formazan. By measuring the absorbance of the formazan over time at the wavelength near the maximum absorption wavelength, and calculating an increase rate of absorbance, NAD can be quantified in the sample.

In the embodiment, an NAD measurement reagent included in a kit preferably also includes a standard solution comprising NAD at a predetermined concentration (NAD standard solution). A calibration curve can be prepared from an increase rate of absorbance obtained by performing the similar reaction as the sample with the NAD standard solution. When the NAD standard solution is used, the NAD standard solution preferably comprises nicotinamide and/or nicotinamide derivative for stabilizing NAD, in the same manner as the composition according to section "3. NAD stabilizing compositions."

As needed, a pH buffer such as phosphoric acid, citric acid, Tris, MES, HEPES, or PIPES, a chelating agent such as EDTA, a surfactant, an antioxidant, an antiseptic, or the like may be added to the first test solution, the second test solution and the NAD standard solution as needed.

The pH of the second test solution is preferably 7.5 to 9.0, particularly 7.5 to 8.5, to stabilize diaphorase. Conversely, the pH of the first test solution is preferably 5.0 to 7.4, particularly 6.0 to 7.4.

In the embodiment, in addition to the NAD stabilizing composition and the NAD measurement reagent, the NAD measurement kit may further include a sample storage container that is suitable for a measurement instrument (e.g., 7180 type Hitachi automatic analyzer (manufactured by Hitachi High-Tech Corporation)), instrument operating instructions, and the like.

### Examples

The present invention will be further described in detail below by way of examples. However, the technical scope of the present invention is not limited to the following examples.

### [Reference Example] Stability test of NAD (Recovery test of NAD that is added to human pooled serum)

NAD was added to 2250 µL of human pooled serum to make 50 µmol/L and incubated at refrigeration, 25°C and 37°C. The NAD concentrations in serum were measured at 1, 2, and 19 hours after the start of incubation.

The NAD concentration of the sample (i.e., serum) was measured by the following procedure.

### (1) Deproteinization treatment

Three times the volume of 0.4 M trichloroacetic acid solution was added to the collected sample, mixed, and placed for 10 minutes under a static condition at room temperature. The mixture was then centrifuged, and the supernatant was collected as an NAD extract.

### (2) NAD measurement

NAD in the extract was measured using a 7180 type Hitachi automatic analyzer (manufactured by Hitachi High-Tech Corporation). The measurement was performed by setting the first test solution (comprising a phosphate buffer (pH 7.0), 0.6 mM WST-8 and 1.7 U/mL glucose dehydrogenase (GLUCDH "Amano" 2 manufactured by Amano Enzyme Inc.) and the second test solution (comprising a phosphate buffer (pH 7.8), 320 mM D-glucose and 15 U/mL diaphorase (No. 46445003 manufactured by Oriental Yeast Co., Ltd.) using the measurement parameters as follows.

| | |
|---|---|
| · Analysis method | Rate A |
| · Measurement wavelength (sub/main) | 600nm/450nm |
| · Reaction duration | 10 minutes |
| · Photometric points | 27 to 34 |
| · Sample solution (NAD extract solution) | 2 µL |
| · R1 reagent | 120 µL |
| · R2 reagent | 40 µL |

That is, 2 µL of NAD extract and 120 µL of the first test solution were mixed and incubated at 37°C for 4.5 min (photometric point 1 to 16), and 40 µL of the second test solution was added to start the reaction (photometric point 17). The value of an absorbance change per minute (ΔAbs/min) was calculated by subtracting an absorbance of water (blank) at the same photometric point from the absorbance value of the measured sample at 8 to 10 minutes (photometric point 27 to 34) after the start of reaction.

The NAD concentration of the sample was calculated from the calibration curve (the NAD concentration - the increase rate of absorbance (ΔAbs/min)) that was obtained beforehand by measuring multiple NAD solutions at predetermined concentrations.

Figure 1 is a graph illustrating a time-course change of relative values (%) of the NAD concentration in serum under each incubation condition when the concentration at the time of NAD addition (0 hour) is set as 100%. When stored at 25°C and 37°C, the NAD concentrations reached almost 0% within two hours. When stored at refrigeration, the NAD concentration was also reduced by about half within two hours and reached to almost 0% within 20 hours. In serum, NAD was shown to be in a highly unstable state.

### [Example 1] Stabilizing study of NAD in blood using an NAD stabilizer (Observation of a time-course change)

Whole blood was collected from SD retired rats using EDTA-filled blood collection tubes. 450 µL of whole blood was transferred to a 1.5-mL centrifuge tube, and 50 µL of a 10 × stabilizer (PBS (pH 6.1) comprising 2 M nicotinamide, 100 mM NMN and 40 mg/mL EDTA) was immediately added and mixed to make a measurement sample. A control sample was prepared by adding PBS instead of the 10 × stabilizer to the whole blood sample. The measurement sample and the control sample were immediately frozen at -80°C and stored. Each frozen sample was thawed at room temperature for approximately 30 minutes, mixed well, and placed under a static condition at refrigeration or 25°C, respectively. The samples at each condition were measured by n = 3.

NAD extraction was performed on the samples after being placed for a predetermined time under a static condition in the same procedure according to (1) in Reference Example. The NAD measurement was performed on each extract in the same procedure according to (2) in Reference Example.

Figure 2 shows results of measuring each sample. Figure 2A is a graph illustrating a time-course change of the NAD concentration in the sample under a static condition at refrigeration. Figure 2B is a graph illustrating a time-course change of the NAD concentration in the sample under a static condition at 25°C. In the control sample, most of the NAD was not detected when the sample was frozen and thawed. The NAD concentration was further decreased by being placed under a static condition at refrigeration and 25°C. On the other hand, in the measurement sample comprising the stabilizer, the NAD concentration hardly decreased even after 24 hours under a static condition at both refrigeration and 25°C, indicating that the NAD in the measurement sample was stable.

### [Example 2] Stabilizing study of NAD in blood using nicotinamide and NMN

Whole blood was collected from SD retired rats using EDTA-filled blood collection tubes. PBS (pH 6.0) comprising nicotinamide and NMN at 10 times the concentration shown in Table 1 was prepared as a 10 × stabilizer for each. 450 µL of whole blood was transferred to a 1.5-mL centrifuge tube, and 50 µL of a 10 × stabilizer for each was added and mixed. NAD was added to each tube to make 100 µM and used as a measurement sample.

The NAD extraction and the NAD measurement were performed on each measurement sample after being placed overnight under a static condition at 25°C, in the same manner according to Example 1. The relative value (%) of NAD concentration of each measured sample was calculated, wherein the concentration at the time of NAD addition (0 hour) was set as 100%. Table 1 shows the results. It was confirmed that the amount of remaining NAD became higher as the amount of nicotinamide and NMN increased.

**[Table 1]**

| | 0 mM NMN | 3 mM NMN | 10 mM NMN | 20 mM NMN |
|---|---|---|---|---|
| 0 mM Nicotinamide | 2.9 | 8.2 | 16.1 | 33.0 |
| 50 mM Nicotinamide | 7.4 | 26.7 | 58.7 | 73.4 |
| 100 mM Nicotinamide | 6.6 | 46.2 | 72.7 | 80.8 |
| 200 mM Nicotinamide | 13.7 | 67.4 | 81.6 | 83.5 |

### [Example 3] Stabilizing study of NAD in blood using nicotinamide and NR

Whole blood was collected from SD retired rats using EDTA-filled blood collection tubes. PBS (pH 6.0) comprising nicotinamide and NR at 10 times the concentration shown in Table 2 was prepared as a 10 × stabilizer for each. 450 µL of whole blood was transferred to a 1.5-mL centrifuge tube, and 50 µL of a 10 × stabilizer for each was added and mixed. NAD was added to each tube to make 100 µM and used as a measurement sample.

The NAD extraction and the NAD measurement were performed on each measurement sample after being placed overnight under a static condition at 25°C, in the same manner according to Example 1. The relative value (%) of NAD concentration of each measured sample was calculated, wherein the concentration at the time of NAD addition (0 hour) was set as 100%. Table 2 shows the results. It was confirmed that the amount of remaining NAD became higher as the amount of nicotinamide and NR increased.

**[Table 2]**

| | 0 mM NR | 3 mM NR | 10 mM NR | 20 mM NR |
|---|---|---|---|---|
| 0 mM Nicotinamide | 2.4 | 7.5 | 49.7 | 64.8 |
| 50 mM Nicotinamide | 7.8 | 7.2 | 62.7 | 79.2 |
| 100 mM Nicotinamide | 8.7 | 6.9 | 68.1 | 83.4 |
| 200 mM Nicotinamide | 10.8 | 18.4 | 78.6 | 83.7 |

### [Example 4] Stabilizing study of NAD in blood using NMN and NR

Whole blood was collected from SD retired rats using EDTA-filled blood collection tubes. PBS (pH 6.0) comprising NMN and NR at 10 times the concentration shown in Table 3 was prepared as a 10 × stabilizer for each. 450 µL of whole blood was transferred to a 1.5-mL centrifuge tube, and 50 µL of 10 × stabilizer for each was added and mixed. NAD was added to each tube to make 100 µM and used as a measurement sample.

The NAD extraction and the NAD measurement were performed on each measurement sample after being placed overnight under a static condition overnight at 25°C, in the same manner according to Example 1. The relative value (%) of NAD concentration of each measured sample was calculated, wherein the concentration at the time of NAD addition (0 hour) was set as 100%. Table 3 shows the results. It was confirmed that the amount of remaining NAD became higher as the amount of NMN and NR increased.

**[Table 3]**

| | 0 mM NMN | 20 mM NMN | 50 mM NMN | 100 mM NMN |
|---|---|---|---|---|
| 0 mM NR | 0.0 | 29.1 | 58.0 | 89.6 |
| 20 mM NR | 62.9 | 69.2 | 72.0 | 87.9 |
| 50 mM NR | 71.2 | 79.1 | 83.8 | 86.0 |
| 100 mM NR | 77.7 | 82.1 | 84.1 | 86.8 |

### [Example 5] Effect of pH on stabilizing NAD in blood

Whole blood was collected from SD retired rats using EDTA-filled blood collection tubes. PBS solution or a citric acid solution comprising 2 M nicotinamide (stabilizer 1) and 100 mM NMN (stabilizer 2) were prepared as a 10 × stabilizer for each, which has respective pH value as shown in Table 4. 450 µL of whole blood was transferred to a 1.5-mL centrifuge tube, and 50 µL of a 10 × stabilizer for each was added and mixed. NAD was added to each tube to make 100 µM and used as a measurement sample.

The NAD extraction and the NAD measurement were performed on each measurement sample after being placed overnight under a static condition at 25°C, in the same manner according to Example 1. The relative value (%) of NAD concentration of each measured sample was calculated, wherein the concentration at the time of NAD addition (0 hour) was set as 100%. Table 4 shows the results. Regarding the pH of the 10 × stabilizer, it was confirmed that a significant difference was not observed in the stabilizing effect of NAD in blood in a range of pH 4.29 to pH 7.11.

**[Table 4]**

| Buffer | Stabilizer 1 (Final concentration in sample) | Stabilizer 2 (Final concentration in sample) | Relative value (%) |
|---|---|---|---|
| PBS pH7.11 | 200 mM Nicotinamide | 10 mM NMN | 73.0 |
| PBS pH6.43 | 200 mM Nicotinamide | 10 mM NMN | 73.8 |
| PBS pH5.54 | 200 mM Nicotinamide | 10 mM NMN | 74.3 |
| PBS pH5.35 | 200 mM Nicotinamide | 10 mM NMN | 74.9 |
| 100 mM Citric acid pH5.40 | 200 mM Nicotinamide | 10 mM NMN | 75.4 |
| 100 mM Citric acid pH4.78 | 200 mM Nicotinamide | 10 mM NMN | 73.0 |
| 100 mM Citric acid pH4.49 | 200 mM Nicotinamide | 10 mM NMN | 73.8 |
| 100 mM Citric acid pH4.29 | 200 mM Nicotinamide | 10 mM NMN | 76.7 |

### Industrial Applicability

The present invention can be used mainly in the industrial fields of clinical laboratory testing and *in vitro* diagnostics.

All publications, patents and patent applications cited herein are hereby incorporated by reference in their entirety.

## Claims

1. A method of stabilizing nicotinamide adenine dinucleotide (NAD) in a sample collected from a subject, comprising a contact step of contacting the sample with nicotinamide and/or nicotinamide derivative.

2. The method according to claim 1, wherein the nicotinamide and/or nicotinamide derivative is nicotinamide, nicotinamide mononucleotide (NMN) and/or nicotinamide riboside (NR).

3. The method according to claim 2, wherein in the contact step:
i) a final concentration of the nicotinamide is 10 to 500 mM;
ii) a final concentration of the NMN is 1 to 100 mM; and/or
iii) a final concentration of the NR is 1 to 100 mM.

4. The method according to claim 1, wherein the sample is whole blood or tissue fluid from the subject.

5. The method according to claim 1, wherein the subject is human.

6. A method of measuring NAD, comprising a step of measuring NAD in a sample stabilized by the method according to any one of claims 1 to 5.

7. The method according to claim 6, further comprising a step of pretreating a sample before the step of measuring NAD.

8. The method according to claim 7, wherein the step of measuring NAD comprises reacting a pretreated sample in a system comprising dehydrogenase with NAD⁺ as a coenzyme and a substrate thereof, diaphorase, and a tetrazolium salt.

9. A composition comprising nicotinamide and/or nicotinamide derivative, for use in stabilizing NAD in a sample.

10. The composition according to claim 9, wherein the nicotinamide and/or nicotinamide derivative is nicotinamide, NMN and/or NR.

11. The composition according to claim 9 or 10, for use in the method according to any one of claims 1 to 5.

12. An NAD measurement kit comprising the composition according to claim 9 or 10 and a reagent for measuring NAD in a sample.
